Europäisches Patentamt

European Patent Office

Office européen des brevets

(19)

(11) **EP 0 791 828 A1**

(12) **EUROPEAN PATENT APPLICATION**

(43) Date of publication:
   **27.08.1997 Bulletin 1997/35**

(51) Int Cl.6: **G01N 27/407**, G01N 27/419

(21) Application number: **97301200.8**

(22) Date of filing: **24.02.1997**

(84) Designated Contracting States:
   **DE FR GB IT SE**

(30) Priority: **23.02.1996 JP 36754/96**
   **07.02.1997 JP 25580/97**

(71) Applicant: **NGK INSULATORS, LTD.**
   **Nagoya-City, Aichi Prefecture 467 (JP)**

(72) Inventors:
   • **Kato, Nobuhide**
   **Ama-gun, Aichi-Pref. 497 (JP)**

   • **Nakagaki, Kunihiko**
   **Nagoya-city, Aichi-pref. 461 (JP)**

(74) Representative: **Paget, Hugh Charles Edward et al**
   **MEWBURN ELLIS**
   **York House**
   **23 Kingsway**
   **London WC2B 6HP (GB)**

(54) **Method for measuring nitrogen oxides**

(57)   Oxygen contained in a measurement gas introduced from external space into a first chamber (54) through a first diffusion rate-determining section (58) is subjected to a pumping process by using a main pumping cell (68) to control a partial pressure of oxygen in the first chamber (54). Oxygen contained in the measurement gas introduced from the first chamber (54) into a second chamber (56) through a second diffusion rate-determining section (60) is subjected to a pumping process by using a measuring pumping cell (84) to control a partial pressure of oxygen in the second chamber (56).

A pumping current (Ip2) flowing through the measuring pumping cell (84) is detected in a state in which the partial pressure of oxygen in the second chamber (56) is not less than the partial pressure of oxygen in the first chamber (54). NOx contained in the measurement gas is measured on the basis of the detected pumping current (Ip2). Accordingly, it is possible to measure the concentration of NOx in the measurement gas in a stable manner for a long period of time in a state in which the offset is always zero, without being affected by, for example, oxygen, $CO_2$, and $H_2O$.

FIG.2

Printed by Jouve, 75001 PARIS (FR)

## Description

BACKGROUND OF THE INVENTION

Field of the Invention:

The present invention relates to a method for measuring nitrogen oxides contained in, for example, atmospheric air and exhaust gas discharged from vehicles or automobiles. Description of the Related Art:

A method for measuring $CO_2$ and $H_2O$ has been hitherto suggested as described in United States Patent No. 4,909,072. Namely, a measurement gas is introduced into a first internal space through a first diffusion rate-determining means. A partial pressure of oxygen in the first internal space is controlled to have a predetermined low value by using an oxygen pump based on the use of a first electrochemical cell. A gas in the first internal space is introduced into a second internal space through a second diffusion rate-determining means to reduce $CO_2$ and $H_2O$ by using an oxygen pump based on the use of a second electrochemical cell provided for the second internal space. Oxygen is produced during the reduction, and the produced oxygen is pumped out. A quantity of electricity is required during this process, on the basis of which $CO_2$ and $H_2O$ are measured.

In the measurement method described above, a voltage applied to the second electrochemical cell provided for the second internal space is made higher than a voltage applied to the first electrochemical cell provided for the first internal space. Thus the oxygen gas is separated from $CO_2$ and $H_2O$ to perform measurement by using a difference in equilibrated partial pressure of oxygen.

However, in the case of the method in which the gas components are separated to perform measurement by the aid of the pumping voltage as described above, it is necessary to apply a high voltage of not less than 0.8 volt to the second electrochemical cell in ordinary cases. Therefore, the electrode is exposed to an excessive reducing atmosphere. As a result, a disadvantage arises in that the pumping ability is gradually decreased due to sintering of the electrode, caused at a low partial pressure of oxygen.

In addition, another disadvantage arises as follows. Namely, when the plus electrode of the second electrochemical cell is exposed to such a reducing atmosphere, its solid electrolyte is reduced, and thus the cell is deteriorated.

European Patent Publication No. 0678740 Al has succeeded in detection of NOx in a separated manner by arranging a catalyst in a second internal space, and applying, to a second electrochemical cell, a voltage of about 450 mV at which $CO_2$ and $H_2O$ are not decomposed.

However, the principle of operation of the conventional methods as described above makes it necessary that the partial pressure of oxygen in the second internal space is lower than the partial pressure of oxygen in the first internal space. A value corresponding to a difference in partial pressure of oxygen between the first and second internal spaces brings about an offset at the zero point of the pumping current flowing through the second electrochemical cell principally corresponding to the amount of NOx. It is feared that such an offset may increase an error especially when a minute amount of NOx is measured.

SUMMARY OF THE INVENTION

The present invention aims to overcome or to ameliorate the disadvantages involved in the conventional methods for measuring nitrogen oxides as described above and aims to provide a method for measuring nitrogen oxides, which makes it possible to measure, for example, the concentration of NOx in a measurement gas in a stable manner for a long period of time in a state in which the offset is always zero, without being affected by, for example, oxygen, $CO_2$, and $H_2O$.

According to the present invention a method for measuring nitrogen oxides, is provided comprising the steps of using a main pumping means including a pair of first and second pumping electrodes, the first pumping electrode being arranged in an area into which a measurement gas is introduced from external space, wherein oxygen contained in the measurement gas introduced from the external space is subjected to a pumping process on the basis of a control voltage applied between the pair of pumping electrodes so that a partial pressure of oxygen in a process atmosphere is controlled to have a predetermined value at which NO is not decomposable; using an electric signal-generating conversion means including a pair of first and second detecting electrodes, the first detecting electrode being arranged in an area into which the measurement gas is introduced after being subjected to the pumping process performed by the main pumping means, wherein the electric signal-generating conversion means provides, by conversion, an electric signal corresponding to an amount of oxygen contained in the measurement gas after being subjected to the pumping process performed by the main pumping means; and measuring the nitrogen oxides in the measurement gas on the basis of the electric signal supplied from the electric signal-generating conversion means.

In the present invention, the nitrogen oxides are preferably measured by operating the electric signal-generating conversion means as follows.

Namely, the oxygen contained in the measurement gas after being subjected to the pumping process performed by the main pumping means is subjected to a pumping process on the basis of a measuring voltage applied between the pair of detecting electrodes so that a partial pressure of oxygen in a process atmosphere is controlled to have a predetermined value at which NO is decomposable; a pumping current flowing through the electric signal-generating conversion means in accordance with the pumping process performed by the electric signal-generating conversion means is detected in a state in which the partial pressure of oxygen in the process atmosphere processed by the electric signal-generating conversion means is not less than the partial pressure of oxygen in the process atmosphere processed by the main pumping means; and the nitrogen oxides in the measurement gas are measured on the basis of the detected pumping current.

Alternatively, the electric signal-generating conversion means is operated as follows. Namely, the oxygen contained in the measurement gas after being subjected to the pumping process performed by the main pumping means is subjected to a pumping process on the basis of a measuring voltage applied between the pair of detecting electrodes so that a partial pressure of oxygen in a process atmosphere is controlled to have a predetermined value at which NO is decomposable; a pumping current flowing through the electric signal-generating conversion means in accordance with the pumping process performed by the electric signal-generating conversion means is detected in a state in which a level of the measuring voltage supplied to the electric signal-generating conversion means is not more than a level of the control voltage supplied to the main pumping means; and the nitrogen oxides in the measurement gas are measured on the basis of the detected pumping current.

In the method according to the present invention, a pumping voltage sufficient to decompose the nitrogen oxides is applied between the pair of detecting electrodes, and/or the measuring pumping means is arranged with a nitrogen oxide-decomposing catalyst for decomposing the nitrogen oxides. In such an embodiment, oxygen is produced from the nitrogen oxides decomposed by the action of the pumping voltage and/or the action of the nitrogen oxide-decomposing catalyst. The produced oxygen is subjected to the pumping process. During this process, a pumping current flows through the electric signal-generating conversion means, corresponding to an amount of oxygen subjected to the pumping process. Therefore, the nitrogen oxides corresponding to the amount of oxygen can be measured by detecting the pumping current flowing through the electric signal-generating conversion means.

Especially, in the present invention, the pumping current flowing through the electric signal-generating conversion means is detected in the state in which the partial pressure of oxygen in the process atmosphere processed by the electric signal-generating conversion means is not less than the partial pressure of oxygen in the process atmosphere processed by the main pumping means. Alternatively, the pumping current flowing through the electric signal-generating conversion means is detected in the state in which the level of the measuring voltage is not more than the level of the control voltage. Accordingly, the offset of the pumping current flowing through the electric signal-generating conversion means can be made zero. Thus even a minute amount of the nitrogen oxides can be correctly measured. Moreover, when the partial pressure of oxygen in the process atmosphere processed by the electric signal-generating conversion means is made higher than the partial pressure of oxygen in the process atmosphere processed by the main pumping means, the pair of detecting electrodes of the electric signal-generating conversion means can be prevented from sintering.

Namely, in the case of the conventional measurement method, the concentration Cn of NOx to be measured resides in $Cn = K \cdot Ip2 - A$ concerning the pumping current Ip2 to be pumped out by the second electrochemical cell, wherein K represents a constant, and A represents an offset value caused by remaining oxygen in the first internal space.

On the contrary, according to the measurement method of the present invention, the control is performed to achieve the state in which the partial pressure of oxygen in the process atmosphere processed by the electric signal-generating conversion means is not less than the partial pressure of oxygen in the process atmosphere processed by the main pumping means. Alternatively, the level of the measuring voltage is controlled to be not more than the level of the control voltage. Therefore, the pumping current flowing through the electric signal-generating conversion means does not contain any pumping current corresponding to remaining oxygen in the process atmosphere processed by the main pumping means. With reference to the relational expression described above, the offset value A is zero in accordance with the principle, and there is given $Cn = K \cdot Ip2$. Therefore, according to the measurement method of the present invention, the accuracy is increased especially when NOx in a high oxygen concentration, or a minute amount of NOx is measured.

In addition, an effect is obtained in that the measurement can be performed highly accurately even when the measurement gas from the external space has an $O_2$-rich atmosphere as described below.

Namely, the diffusion resistance exerted on $O_2$ differs between the $O_2$-rich atmosphere and the $O_2$-lean atmosphere. In the $O_2$-lean atmosphere, no excessive oxygen is pumped out because oxygen is pumped out by the aid of the main pumping means. However, in general, in the $O_2$-rich atmosphere, excessive oxygen is pumped into because the pumping process is performed with respect to inexhaustible oxygen supply sources ($H_2O$, $CO_2$) in a state of no diffusion resistance.

However, in the present invention, the partial pressure of oxygen in the process atmosphere processed by the main pumping means is made to be equivalent to, or not more than the partial pressure of oxygen in the process atmosphere processed by the electric signal-generating conversion means. Accordingly, even when the measurement gas from the external space has an

$O_2$-rich atmosphere, it is enough to deal with a small amount of oxygen pumped into the process space concerning the main pumping means, resulting in an advantage from a viewpoint of detection accuracy.

According to the measurement method of the present invention, the nitrogen oxides are also preferably measured by operating the electric signal-generating conversion means as follows.

Namely, the electric signal-generating conversion means may be operated such that an electromotive force corresponding to a difference between the amount of oxygen contained in the measurement gas after being subjected to the pumping process performed by the main pumping means and an amount of oxygen contained in a gas existing on a side of the second detecting electrode is detected in a state in which the partial pressure of oxygen in the process atmosphere processed by the electric signal-generating conversion means is not less than the partial pressure of oxygen in the process atmosphere processed by the main pumping means; and the nitrogen oxides in the measurement gas are measured on the basis of the detected electromotive force.

In a preferred embodiment concerning the electric signal-generating conversion means, the electric signal-generating conversion means may be arranged with a nitrogen oxide-decomposing catalyst for decomposing the nitrogen oxides. In such an arrangement, the electric signal-generating conversion means generates, between the pair of the detecting electrodes, an electromotive force of an oxygen concentration cell corresponding to a difference between an amount of oxygen produced from the nitrogen oxides decomposed by the action of the nitrogen oxide-decomposing catalyst and the amount of oxygen contained in the gas existing on the side of the second detecting electrode. Thus the nitrogen oxides, which correspond to the amount of oxygen, are measured by detecting the electromotive force.

In the measurement method described above, the electromotive force generated in the electric signal-generating conversion means is detected in the state in which the partial pressure of oxygen in the process atmosphere processed by the electric signal-generating conversion means is not less than the partial pressure of oxygen in the process atmosphere processed by the main pumping means as well. Accordingly, the offset of the electromotive force, which would be otherwise generated in the electric signal-generating conversion means, can be made zero. Thus even a minute amount of the nitrogen oxides can be correctly measured. Especially, the accuracy is increased when NOx in a high oxygen concentration, or a minute amount of NOx is measured. Moreover, when the partial pressure of oxygen in the process atmosphere processed by the electric signal-generating conversion means is higher than the partial pressure of oxygen in the process atmosphere processed by the main pumping means, the pair of detecting electrodes of the electric signal-generating conversion means can be prevented from sintering.

In the measurement method described above, the partial pressure of oxygen in the process atmosphere processed by the main pumping means is made to be equivalent to, or not more than the partial pressure of oxygen in the process atmosphere processed by the electric signal-generating conversion means as well. Accordingly, even when the measurement gas from the external space has an $O_2$-rich atmosphere, it is enough to deal with a small amount of oxygen pumped into the process space concerning the main pumping means, resulting in an advantage from a viewpoint of detection accuracy.

In the measurement method according to the present invention, it is preferable that an electrode having a low catalytic activity on NO is used as a least one of the electrodes exposed to spaces for processing the measurement gas introduced from the external space. In this embodiment, the electrode has an extremely low activity as a nitrogen oxide-decomposing catalyst, and it does not decompose NO even at a low partial pressure of oxygen. Therefore, the oxygen, which serves as a disturbing component for the measurement of the nitrogen oxides, can be eliminated to be substantially zero, without exerting any influence on the measurement of the nitrogen oxide. As a result, the nitrogen oxides contained in the measurement gas can be measured highly accurately and stably by the aid of the electric signal-generating conversion means.

In the measurement method described above, it is desirable that an electrode having a low catalytic activity on NO contains Au or alloy composed of Au and element belonging to the platinum group. In this embodiment, it is possible to more preferably avoid the decomposing action on NO on the electrode exposed to the process atmosphere processed by the main pumping means.

The measurement method described above may further comprise the steps of using a concentration-detecting means including a pair of first and second measuring electrodes, the first measuring electrode being arranged to be opposed to the first pumping electrode of the main pumping means, wherein an electromotive force of an oxygen concentration cell generated corresponding to a difference between an amount of oxygen contained in the measurement gas during the pumping process performed by the main pumping means and an amount of oxygen contained in a gas existing on a side of the second measuring electrode is measured; and adjusting the control voltage for the main pumping means on the basis of the electromotive force measured by the concentration-measuring means.

Accordingly, the electromotive force generated corresponding to the difference between the amount of oxygen contained in the measurement gas during the pumping process performed by the main pumping means and the amount of oxygen contained in the gas existing on the side of the second measuring electrode is measured by the concentration-measuring means.

The level of the control voltage applied between the pair of pumping electrodes of the main pumping means is adjusted on the basis of the measured electromotive force.

The main pumping means performs the pumping process for an amount of the oxygen in the measurement gas introduced from the external space, the amount corresponding to the level of the control voltage. The oxygen concentration in the measurement gas is subjected to feedback control so that the oxygen concentration is at a predetermined level by supplying, to the main pumping means, the control voltage having been subjected to the adjustment for the level as described above.

The measurement method described above may further comprise the step of using an auxiliary pumping means including an auxiliary pumping electrode provided in the vicinity of the first detecting electrode, wherein the oxygen contained in the measurement gas after being subjected to the pumping process performed by the main pumping means is subjected to a pumping process on the basis of a voltage applied between the auxiliary pumping electrode and the second detecting electrode.

Accordingly, the measurement gas, which has been firstly subjected to the coarse adjustment so that the predetermined gas component has a predetermined concentration by the aid of the main pumping means, is further subjected to the fine adjustment for the concentration of the predetermined gas component by the aid of the auxiliary pumping means.

In general, when the concentration of the predetermined gas component in the measurement gas in the external space greatly changes (for example, from 0 to 20 %), the concentration distribution of the predetermined gas component in the measurement gas introduced into the main pumping means greatly changes. The amount of the predetermined gas component introduced into the electric signal-generating conversion means also changes.

In such a situation, the oxygen concentration in the measurement gas after being subjected to the pumping process performed by the main pumping means is finely adjusted upon the pumping process performed by the auxiliary pumping means. Significantly, the change in oxygen concentration in the measurement gas introduced into the auxiliary pumping means is greatly reduced as a result of the pumping process performed by the main pumping means, as compared with the change in oxygen concentration in the measurement gas supplied from the external space (the measurement gas introduced into the main pumping means). Accordingly, the concentration of the predetermined gas component can be accurately and constantly controlled in the vicinity of the first detecting electrode of the electric signal-generating conversion means.

Therefore, the concentration of the predetermined gas component introduced into the electric signal-generating conversion means is scarcely affected by the change in oxygen concentration in the measurement gas (the measurement gas introduced into the main pumping means). As a result, the pumping current value or the electromotive force detected as described above is not affected by the change in concentration of the predetermined gas component in the measurement gas. Thus a value is obtained, which accurately corresponds to the amount of the objective component existing in the measurement gas.

In the measurement method according to the present invention, the second measuring electrode of the concentration-measuring means is arranged at a position exposed to a space into which a reference gas is introduced. Thus it is possible to compare the oxygen contained in the measurement gas with the oxygen contained in the reference gas, making it possible to detect the nitrogen oxides more correctly.

Especially, it is preferable to combine the second measuring electrode and the second detecting electrode of the electric signal-generating conversion means into a common unit. In this embodiment, a common electrode, which serves as both of the second measuring electrode of the concentration-measuring means and the second detecting electrode of the electric signal-generating conversion means, is exposed to the reference gas-introducing space. The common electrode can be defined as a reference electrode for the respective detecting processes performed by the concentration-measuring means and the electric signal-generating conversion means. In accordance with this definition, the first measuring electrode of the concentration-measuring means, and the first detecting electrode of the electric signal-generating conversion means can be defined as a measuring electrode and a detecting electrode respectively.

The main pumping means may include inner and outer pumping electrodes formed at the inside and the outside of a first chamber surrounded by substrates composed of solid electrolytes, for introducing the measurement gas thereinto, and the substrate interposed between the both electrodes. The electric signal-generating conversion means may include a detecting electrode formed at the inside of a second chamber surrounded by substrates composed of solid electrolytes, for introducing the measurement gas thereinto after being subjected to the pumping process performed by the main pumping means, a reference electrode formed at the inside of a reference gas-introducing space surrounded by substrates composed of solid electrolytes, for introducing a reference gas thereinto, and the substrate interposed between the detecting electrode and the reference electrode.

The concentration-measuring means may include a measuring electrode formed at the inside of a first chamber surrounded by substrates composed of solid electrolyte, for introducing the measurement gas from the external space, a reference electrode formed at the inside of a reference gas-introducing space surrounded by substrates composed of solid electrolytes, for introducing a reference gas thereinto, and the substrate interposed between the measuring electrode and the reference electrode.

Preferably, in the measurement method described above, the measurement gas is introduced from the external space into the first chamber through a first diffusion rate-determining section for giving a predetermined diffusion resistance to the measurement gas, and the measurement gas after being subjected to the pumping process performed by the main pumping means is introduced into the second chamber through a second diffusion rate-determining section for giving a predetermined diffusion resistance to the measurement gas.

In a preferred embodiment, the measurement gas in the second chamber may be introduced into an area of the first detecting electrode through a third diffusion rate-determining section for giving a predetermined diffusion resistance to the measurement gas.

Preferably, the solid electrolyte is an oxygen ion-conductive solid electrolyte based on the use of a ceramic such as $ZrO_2$. Preferably, a porous material for giving a predetermined resistance to the measurement gas is used for the first or second diffusion rate-determining section in order to achieve a designed desired state for the measurement gas in the first and second chambers.

Preferably, an Rh cermet is used for the nitrogen oxide-decomposing catalyst for constructing the electrode or the catalyst arranged in the first and second chambers.

The above and other optional features and advantages of the present invention will become more apparent from the following description when taken in conjunction with the accompanying drawings in which embodiments of the present invention are shown by way of illustrative example.

DESCRIPTION OF THE DRAWINGS

FIG. 1 shows a plan view illustrating a sensing device according to a first embodiment.

FIG. 2 shows a cross-sectional view taken along a line A-A in FIG. 1.

FIG. 3 shows a cross-sectional view illustrating a modified embodiment of the sensing device according to the first embodiment.

FIG. 4 shows a cross-sectional view illustrating a sensing device according to a second embodiment.

FIG. 5 shows a graph illustrating output characteristics of a pumping current Ip2 flowing through a measuring pumping cell obtained by using a pumping current Vpl of 450 mV, and a measuring voltage Vp2 of 400 mV or 600 mV.

FIG. 6 shows a cross-sectional view illustrating a modified embodiment of the sensing device according to the second embodiment.

FIG. 7 shows a cross-sectional view illustrating a sensing device according to a third embodiment.

FIG. 8 shows a graph illustrating an output characteristic of a pumping current Ip2 flowing through a measuring pumping cell obtained by using a partial pressure of oxygen in a first chamber of $10^{-8}$ atm, and a partial pressure of oxygen in a second chamber 56 (interface between a second diffusion rate-determining section and a detecting electrode) of $10^{-6}$ atm.

FIG. 9 shows a cross-sectional view illustrating a modified embodiment of the sensing device according to the third embodiment.

FIG. 10 shows a cross-sectional view illustrating a sensing device according to a fourth embodiment.

FIG. 11 shows a cross-sectional view illustrating a modified embodiment of the sensing device according to the fourth embodiment.

FIG. 12 shows a cross-sectional view illustrating a sensing device according to a fifth embodiment.

FIG. 13 shows a cross-sectional view illustrating a modified embodiment of the sensing device according to the fifth embodiment.

DESCRIPTION OF THE EMBODIMENTS

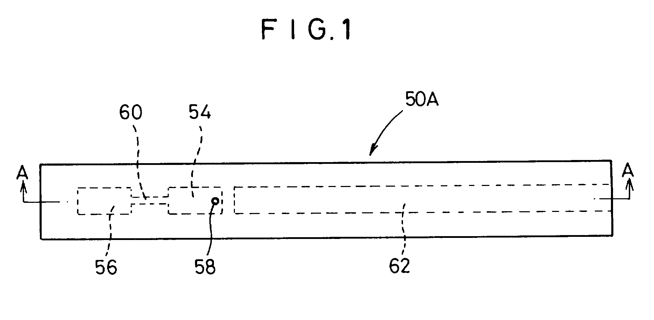

Several embodiments, in which the method for measuring nitrogen oxides according to the present invention is applied to sensing devices for measuring nitrogen oxides such as NO and $NO_2$ contained in, for example, atmospheric air or exhaust gas discharged from vehicles or automobiles, will be explained below with reference to FIGs. 1 to 13.

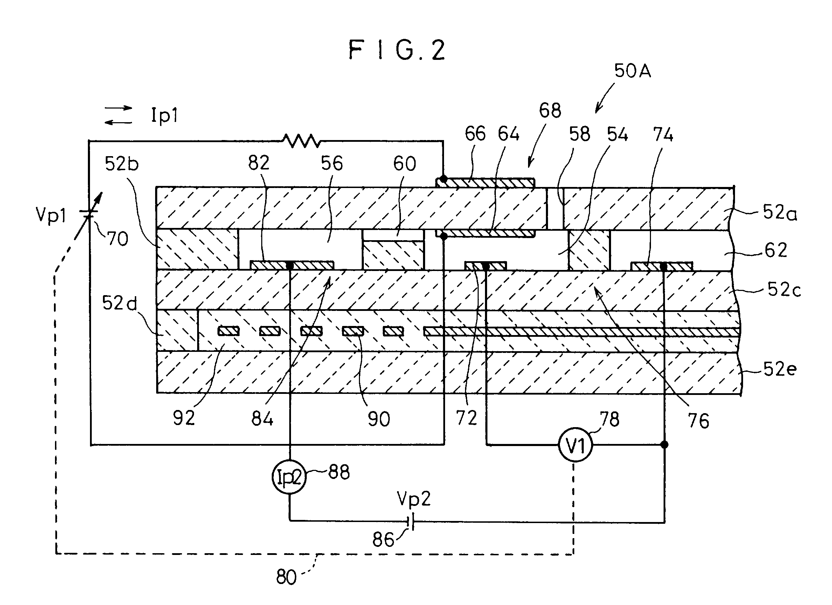

At first, as shown in FIGs. 1 and 2, a sensing device 50A according to a first embodiment has a lengthy plate-shaped configuration as a whole, comprising, for example, five stacked solid electrolyte layers 52a to 52e composed of ceramics based on the use of oxygen ion-conductive solid electrolytes such as $ZrO_2$. A first layer from the bottom is designated as a substrate layer 52e. Second and fourth layers from the bottom are designated as first and second spacer layers 52d, 52b respectively. Third and fifth layers from the bottom are designated as first and second solid electrolyte layers 52c, 52a respectively.

Specifically, the first spacer layer 52d is stacked on the substrate layer 52e. The first solid electrolyte layer 52c, the second spacer layer 52b, and the second solid electrolyte layer 52a are successively stacked on the first spacer layer 52d.

A first chamber 54 for adjusting the partial pressure of oxygen in a measurement gas, and a second chamber 56 for finely adjusting the partial pressure of oxygen in the measurement gas and measuring oxides, for example, nitrogen oxides (NOx) in the measurement gas are formed and comparted by a lower surface of the second solid electrolyte layer 52a, side surfaces of the second spacer layer 52b, and an upper surface of the first solid electrolyte layer 52c.

A through hole (first diffusion rate-determining section) 58 for communicating the first chamber 54 with an external measurement gas-existing space is provided at a portion of the second solid electrolyte layer 52a corresponding to the first chamber 54.

The second spacer layer 52b is interposed at a front end of the sensing device 50A between the first and second solid electrolyte layers 52c, 52a. A second diffusion rate-determining section 60 is interposed between the first and second chambers 54, 56.

A space for introducing a reference gas, for example, atmospheric air to serve as a reference for oxide measurement (reference gas-introducing space 62) is formed and comparted by the lower surface of the second solid electrolyte layer 52a, side surfaces of the second spacer layers 52b, and the upper surface of the first solid electrolyte layer 52c

Namely, the sensing device 50A according to the first embodiment has a configuration in which all of the first chamber 54, the second chamber 56, and the reference gas-introducing space 62 are formed at the stacked position of the second spacer layer 52b, and they are arranged on a substantially identical plane.

The first and second diffusion-rate determining sections 58, 60 give predetermined diffusion resistances to the measurement gas to be introduced into the first and second chambers 54, 56 respectively. Each of the first and second diffusion-rate determining sections 54, 56 can be formed as a passage composed of, for example, a porous material, or a small hole having a predetermined cross-sectional area so that the measurement gas may be introduced.

Preferably, the second diffusion-rate determining section 60 is arranged and filled with a porous material comprising, for example, $ZrO_2$ so that the diffusion resistance of the second diffusion-rate determining section 60 is made larger than the diffusion resistance of the first diffusion-rate determining section 58.

An inner pumping electrode 64, which is composed of a porous cermet electrode having a flat and substantially rectangular shape, is formed on a surface portion for forming the first chamber 54, of the lower surface of the second solid electrolyte layer 52a. An outer pumping electrode 66 is formed on a portion corresponding to the inner pumping electrode 64, of the upper surface of the second solid electrolyte layer 52a. An electrochemical pumping cell, i.e., a main pumping cell 68 is constructed by the inner pumping electrode 64, the outer pumping electrode 66, and the second solid electrolyte layer 52a interposed between the both electrodes 64, 66.

A desired control voltage (pumping voltage) Vpl is applied between the inner pumping electrode 64 and the outer pumping electrode 66 of the main pumping cell 68 by the aid of an external variable power source 70 to allow a pumping current Ipl to flow in a positive or negative direction between the outer pumping electrode 66 and the inner pumping electrode 64. Thus the oxygen in the atmosphere in the first chamber 54 can be pumped out to the external space, or the oxygen in the external space can be pumped into the first chamber 54.

A measuring electrode 72, which is composed of a porous cermet electrode having a flat and substantially rectangular shape, is formed on a portion adjacent to the second diffusion rate-determining section 60, of the upper surface of the first solid electrolyte layer 52c for forming the first chamber 54. A reference electrode 74 is formed on a portion exposed to the reference gas-introducing space 62, of the lower surface of the first solid electrolyte layer 52c. An electrochemical sensor cell, i.e., a controlling oxygen partial pressure-detecting cell 76 is constructed by the measuring electrode 72, the reference electrode 74, and the first solid electrolyte layer 52c.

An electromotive force is generated between the measuring electrode 72 and the reference electrode 74 of the controlling oxygen partial pressure-detecting cell 76 on the basis of a difference in oxygen concentration between the atmosphere in the first chamber 54 and the reference gas (atmospheric air) in the reference gas-introducing space 62. The partial pressure of oxygen in the atmosphere in the first chamber 54 can be detected by measuring the generated electromotive force by the aid of a voltmeter 78.

Namely, the voltage V1, which is generated between the reference electrode 74 and the measuring electrode 72, is an electromotive force of the oxygen concentration cell, generated on the basis of a difference between a partial pressure of oxygen in the reference gas introduced into the reference gas-introducing space 62 and a partial pressure of oxygen in the measurement gas in the first chamber 54. The voltage V1 has the following relationship known as the Nernst's equation.

$$V1 = RT/4F \cdot \ln(P1(O_2)/P0(O_2))$$

R:          gas constant;
T:          absolute temperature;
F:          Faraday constant;
$P1(O_2)$):    partial pressure of oxygen in the first chamber 54;
$P0(O_2)$):    partial pressure of oxygen in the reference gas.

Therefore, the partial pressure of oxygen in the first chamber 54 can be detected by measuring the voltage V1 generated on the basis of the Nernst's equation, by the aid of the voltmeter 78.

The detected value of the partial pressure of oxygen is used to control the pumping voltage of the variable power source 70 by the aid of a feedback control system 80. Specifically, the pumping operation performed by the main pumping cell 68 is controlled so that the partial pressure of oxygen in the first chamber 54 has a predetermined value which is sufficiently low to control the partial pressure of oxygen in the second chamber 56 in the next step.

The inner pumping electrode 64 and the outer pumping electrode 66 are composed of an inactive material having a low catalytic activity on NOx, for example, NO in the measurement gas introduced in the first chamber 54. Specifically, the inner pumping electrode 64 and the outer pumping electrode 66 can be composed of a porous cermet electrode. In this embodiment, they are formed of a metal such as Pt and a ceramic such as $ZrO_2$. Especially, it is necessary, for the inner pumping electrode 64 and the measuring electrode 72 arranged in the first chamber 54 contacting with the measurement gas, to use a material having a weak reducing ability or no reducing ability with respect to the NO components in the measurement gas. It is preferable that the inner pumping electrode 64 and the measuring electrode 72 are composed of, for example, a compound having the perovskite structure such as $La_3CuO_4$, a cermet comprising a ceramic and a metal having a low catalytic activity such as Au, or a cermet comprising a ceramic, a metal of the Pt group, and a metal having a low catalytic activity such as Au. Further, when an alloy comprising Au and a metal of the Pt group is used as an electrode material, it is preferable to add Au in an amount of 0.03 to 35 vol% of the entire metal components.

As shown in FIG. 2, the sensing device 50A according to the first embodiment includes a detecting electrode 82 composed of a porous cermet electrode having a flat and substantially rectangular shape. The detecting electrode 82 is formed on a portion separated from the second diffusion rate-determining section 60, of the upper surface of the first solid electrolyte layer 52c for forming the second chamber 56. An electrochemical pumping cell, i.e., a measuring pumping cell 84 is constructed by the detecting electrode 82, the reference electrode 74, and the first solid electrolyte layer 52c.

The detecting electrode 82 can be constructed by appropriately selecting a nitrogen oxide-decomposing catalyst, for example, an Rh cermet, a material having a low catalytic activity, or a combination of a nitrogen oxide-decomposing catalyst arranged in the vicinity of a material having a low catalytic activity.

In the first embodiment, the detecting electrode 82 is composed of a porous cermet comprising Rh as a metal capable of reducing NOx as the measurement gas component, and zirconia as a ceramic. Thus the detecting electrode 82 functions as a NOx-reducing catalyst for reducing NOx existing in the atmosphere in the second chamber 56. Moreover, the oxygen in the atmosphere in the second chamber 56 can be pumped out to the reference gas-introducing chamber 62 by applying a constant voltage Vp2 between the detecting electrode 82 and the reference electrode 74 by the aid of a DC power source 86. A pumping current Ip2, which is allowed to flow in accordance with the pumping operation performed by the measuring pumping cell 84, is detected by an ammeter 88.

The sensing device 50A according to the first embodiment includes a heater 90 for generating heat in accordance with electric power supply from the outside. The heater 90 is embedded in a form of being interposed between the first solid electrolyte layer 52c and the substrate layer 52e and surrounded by the first spacer layer 52d on its three sides. The heater 90 is provided in order to increase the conductivity of oxygen ion. A ceramic layer 92 composed of alumina or the like is formed to cover upper and lower surfaces of the heater 90 so that the heater 90 is electrically insulated from the substrate layer 52e and the first solid electrolyte layer 52c.

As shown in FIG. 2, the heater 90 is arranged with deviation toward the second chamber 56 located on the front end side of the sensing device 50A so that the second chamber 56 is heated to a higher temperature as compared with the first chamber 54, in other words, the detecting electrode 82 is heated to a higher temperature as compared with the inner pumping electrode 64 and the measuring electrode 72.

The heater 90 is arranged such that when the temperature of the measurement gas changes, for example, in a range of 300 °C to 850 °C, the inner pumping electrode 64 and the measuring electrode 72 in the first chamber 54 are heated to 400 °C to 900 °C, and the detecting electrode 82 in the second chamber 56 is heated to 700 °C to 900 °C. The object of such an arrangement is that the oxygen ion-conductivity of the solid electrolyte layer is maintained to have a predetermined value, the polarization of the electrode is minimized, and the activity of the catalyst is maintained.

Especially, the sensing device 50A according to the first embodiment is constructed such that the pumping current Ip2 flowing through the measuring pumping cell 84 is detected in a state in which the partial pressure of oxygen in the second chamber 56 is higher than, or equal to the partial pressure of oxygen in the first chamber 54. Specifically, the pumping current Ip2 flowing through the measuring pumping cell 84 is detected by the aid of the ammeter 88 in a state in which the voltage Vp2 of the DC power source 86 for the measuring pumping cell 84 is lower than, or equal to the pumping voltage Vp1 for the main pumping cell 68.

The sensing device 50A according to the first embodiment is basically constructed as described above. Next, its function and effect will be explained.

Before the measurement of oxides, the sensing device 50A according to the first embodiment is set in a state in which the measurement gas can be introduced into the first chamber 54. Next, an electric power is applied to the heater 90 so that the first and second solid electrolyte layers 52c, 52a for the first chamber 54 of the sensing device 50A are heated to, for example, 400 °C to 900 °C, and the first and second solid electrolyte layers 52c, 52a for the second chamber 56 are heated to, for example, 700 °C to 900 °C. After the sensing device 50A is heated in such a temperature state, the first and second solid electrolyte layers 52c, 52a are activated in a desired state.

Next, the measurement of oxides such as NOx contained in the measurement gas is started by introducing the measurement gas into the sensing device 50A having been set as described above. The measurement gas is introduced into the first chamber 54 under a predetermined diffusion resistance through the first diffusion rate-determining section 58. The partial pressure of oxygen contained in the measurement gas is controlled to have a predetermined value in accordance with a predetermined pumping voltage Vp1 applied between the outer pumping electrode 66 and the inner pumping electrode 64 by the aid of the variable power source 70. Namely, the partial pressure of oxygen in the first chamber 54 can be measured on the basis of a voltage V1 between the reference electrode 74 and the measuring electrode 72, detected by the voltmeter 78.

The voltage V1 is an electromotive force of the oxygen concentration cell defined by the Nernst's equation described above. The pumping voltage Vp1 applied by the variable power source 70 is controlled by the aid of the feedback control system 80 so that the voltage V1 is, for example, 203 mV (500 °C). Thus the partial pressure of oxygen in the first chamber 54 is controlled to be, for example, $10^{-6}$ atm. The first diffusion rate-determining section 58 functions to limit the amount of oxygen in the measurement gas diffusing and flowing into the measuring space (first chamber 54) so that the pumping current Ip1 flowing through the main pumping cell 68 is suppressed when the pumping voltage Vp1 is applied between the inner pumping electrode 64 and the outer pumping electrode 66 of the main pumping cell 68.

A state of the partial pressure of oxygen is formed in the first chamber 54, in which NO in the atmosphere is not decomposed by the inner pumping electrode 64 and the measuring electrode 72, i.e., a condition of the partial pressure of oxygen is formed in the first chamber 54, under which, for example, the reaction of NO → $1/2N_2 + 1/2O_2$ is not caused, even under an environment brought about by being heated by the external measurement gas, and being heated by the heater 90.

This is because of the following reason. Namely, if NO in the measurement gas (atmosphere) is decomposed into $N_2$ and $O_2$ in the first chamber 54, NOx cannot be measured correctly in the second chamber 56. In this context, it is necessary to form a condition under which NO is not decomposed by any component which participates in decomposition of NO in the first chamber 54 (at least the component of the inner pumping electrode 64 of the main pumping cell 68).

The measurement gas, which has been controlled to have the predetermined partial pressure of oxygen in the first chamber 54, is introduced into the second chamber 56 through the second diffusion rate-determining section 60 designed to have a diffusion resistance larger than that of the first diffusion rate-determining section 58.

The NOx components in the measurement gas introduced into the second chamber 56 undergo the action of the NOx-decomposing catalyst arranged in the second chamber 56, and they are decomposed into nitrogen and oxygen. The oxygen produced in this process is subjected to the pumping action performed by the measuring pumping cell 84, in which a predetermined voltage, for example, 185 mV (600 °C) is applied between the detecting electrode 82 and the reference electrode 74 of the measuring pumping cell 84 in a direction to pump out the oxygen from the second chamber 56 to the reference gas-introducing space 62.

Accordingly, the oxygen produced by the decomposition of NOx is pumped out to the outside of the second chamber 56, and the partial pressure of oxygen in the second chamber 56 becomes, for example, $10^{-5}$ atm. At this moment, the pumping current Ip2 flowing through the measuring pumping cell 84 is within an amount of the oxygen produced by the decomposition in the second chamber 56. Any current based on any oxygen remained in the first chamber 56 is not added to the pumping current Ip2.

Namely, the sensing device 50A according to the first embodiment makes it possible to eliminate the influence of a minute amount of oxygen remaining in the first chamber 54 which would otherwise cause the offset of the sensor output as in the conventional method. Thus NOx can be detected with a high degree of accuracy.

It is assumed, for example, that the oxygen concentration in the atmosphere in the first chamber 54 is 0.02 ppm, the oxygen concentration in the atmosphere in the second chamber 56 is 0.2 ppm, and the NO concentration in the measurement gas is 100 ppm. Under this condition, the pumping current Ip2 flows in an amount corresponding to a difference = 49.8 ppm between the oxygen concentration of 50 ppm produced by decomposition of NO and the oxygen concentration of 0.2 ppm in the atmosphere in the second chamber 56. Therefore, almost all of the pumping current value Ip2 concerning the measuring pumping cell 84 represents the amount resulting from the decomposition of NO. Accordingly, the pumping current Ip2 does not depend on the oxygen concentration in the measurement gas.

Namely, almost all of the pumping current Ip2 results from the oxygen produced by the decomposition of the NOx components in the measurement gas. It is possible to accurately measure even a minute amount of NOx in a state in which the influence of oxygen in the measurement gas is eliminated, as compared with the conventional method. It is sufficient that the outer pumping electrode 66 and the reference electrode 74 are formed in an atmosphere to which the oxygen in the first chamber 54 and the second chamber 56 can be released. For example, the outer pumping electrode 66 and the reference electrode 74 may be formed in an atmosphere of air.

As described above, according to the sensing device 50A concerning the first embodiment, the pumping current Ip2 flowing through the measuring pumping cell 84 is detected in the state in which the partial pressure of oxygen in the second chamber 56 is not less than the partial pressure of oxygen in the first chamber 54. Accordingly, the offset of the pumping current Ip2 flowing through the measuring pumping cell 84 can be made zero, and it is possible to accurately measure even a minute amount of NOx. Moreover, when the partial pressure of oxygen in the second chamber 56 is higher than the partial pressure of oxygen in the first chamber 54, the detecting electrode 82 exposed to the atmosphere in the second chamber 56 can be prevented from sintering.

Namely, in the case of the conventional measurement method, the concentration Cn of NOx to be measured satisfies $Cn = K \cdot Ip2 - A$ in relation to the pumping current Ip2 concerning oxygen pumped out by the second electrochemical cell, provided that K represents a constant, and A represents an offset value resulting from remaining oxygen in the first internal space.

On the contrary, in the case of the sensing device 50A according to the first embodiment, the control is performed to achieve the state in which the partial pressure of oxygen in the second chamber 54 is not less than the partial pressure of oxygen in the first chamber 54. Therefore, the pumping current Ip2 flowing through the measuring pumping cell 84 does not contain any pumping current corresponding to remaining oxygen in the first chamber 54. With reference to the relational equation described above, the offset value A is zero in accordance with the principle, substantially giving $Cn = K \cdot Ip2$. Therefore, according to the sensing device 50A concerning the first embodiment, the accuracy is increased especially when NOx in a high oxygen concentration, or a minute amount of NOx is measured.

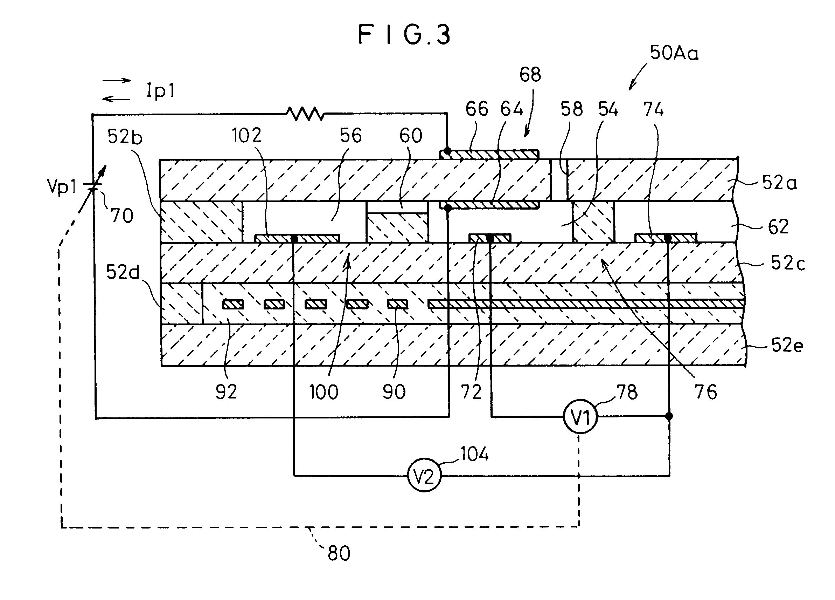

Next, a modified embodiment of the sensing device 50A according to the first embodiment will be explained with reference to FIG. 3. Components or parts corresponding to those shown in FIG. 2 are designated by the same reference numerals, duplicate explanation of which will be omitted.

As shown in FIG. 3, a sensing device 50Aa according to this modified embodiment has approximately the same structure as that of the sensing device 50A according to the first embodiment (see FIG. 2). However, the former is different from the latter in that a measuring oxygen partial pressure-detecting cell 100 is provided in place of the measuring pumping cell 84.

The measuring oxygen partial pressure-detecting cell 100 comprises a detecting electrode 102 formed on a portion for forming the second chamber 56, of the upper surface of the first solid electrolyte layer 52c, the reference electrode 74 formed on the lower surface of the first solid electrolyte layer 52c, and the first solid electrolyte layer 52c.

In this embodiment, an electromotive force (electromotive force of an oxygen concentration cell) V2 is generated between the detecting electrode 102 and the reference electrode 74 of the measuring oxygen partial pressure-detecting cell 100, corresponding to a difference in oxygen concentration between an atmosphere around the detecting electrode 102 and an atmosphere around the reference electrode 74.

Therefore, the partial pressure of oxygen in the atmosphere around the detecting electrode 102, in other words, the partial pressure of oxygen defined by oxygen produced by reduction or decomposition of the measurement gas components (NOx) is detected as a value of the voltage V2 by measuring the electromotive force (voltage) V2 generated between the detecting electrode 102 and the reference electrode 74 by using the voltmeter 104.

When the NO concentration in the external space gradually increases, the reduction or decomposition reaction of NO is caused on the detecting electrode 102, because the detecting electrode 102 also functions as a NOx-reducing catalyst in the same manner as the detecting electrode 82 of the measuring pumping cell 84 described above (see FIG. 2). As a result, the oxygen concentration in an atmosphere around the detecting electrode 102 is increased.

Accordingly, the electromotive force V2 generated between the detecting electrode 102 and the reference electrode 74 is gradually decreased.

The degree of decrease in electromotive force V2 represents the NO concentration. Namely, the electromotive force V2, which is outputted from the measuring oxygen partial pressure-detecting cell 100 constructed by the detecting electrode 102, the reference electrode 74, and the first solid electrolyte layer 52c, represents the NO concentration in the measurement gas.

The electromotive force V2 generated in the measuring oxygen partial pressure-detecting cell 100 is detected in a state in which the partial pressure of oxygen in the second chamber 56 is not less than the partial pressure of oxygen in the first chamber 54 in the sensing device 50Aa according to the modified embodiment as well, in the same manner as the sensing device 50A according to the first embodiment.

Accordingly, the offset of the electromotive force V2 generated in the measuring oxygen partial pressure-detecting cell 100 can be made zero, and it is possible to accurately measure even a minute amount of NOx. Especially, the accuracy is increased when NOx in a high oxygen concentration, or a minute amount of NOx is measured. Moreover, when the partial pressure of oxygen in the second chamber 56 is higher than the partial pressure of oxygen in the first chamber 54, the detecting electrode 102 exposed to the atmosphere in the second chamber 56 can be prevented from sintering.

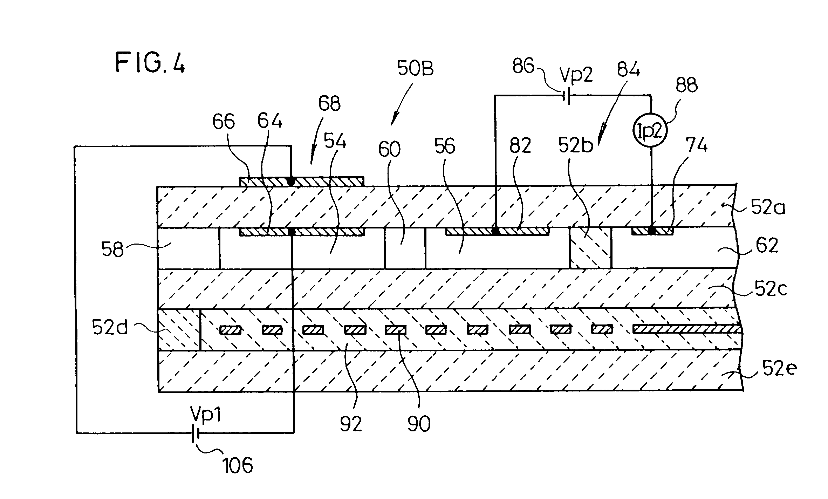

Next, a sensing device 50B according to a second embodiment will be explained with reference to FIG. 4. Components or parts corresponding to those shown in FIG. 2 are designated by the same reference numerals, duplicate explanation of which will be omitted.

As shown in FIG. 4, the sensing device 50B according to the second embodiment has approximately the same structure as that of the sensing device 50A according to the first embodiment. However, the former is different from the latter in the following points.

Namely, the sensing device 50B according to the second embodiment has a simplified structure as compared with the sensing device 50A according to the first embodiment. The controlling oxygen partial pressure-detecting cell 76, which has been provided for the sensing device 50A according to the first embodiment, is omitted. A DC power source 106 is connected between the outer pumping electrode 66 and the inner pumping electrode 64 of the main pumping cell 68.

In the sensing device 50B according to the second embodiment, a first diffusion rate-determining section 58 for communicating the first chamber 54 with the external space is interposed between the first and second solid electrolyte layers 52c, 52a at a front end portion of the sensing device 50B. A first chamber 54 for adjusting the partial pressure of oxygen in the measurement gas is formed and comparted by the lower surface of the second solid electrolyte layer 52a, the side surfaces of the first and second diffusion rate-determining sections 58, 60, and the upper surface of the first solid electrolyte layer 52c. A second chamber 56 for finely adjusting the partial pressure of oxygen in the measurement gas and measuring oxides, such as nitrogen oxides (NOx) in the measurement gas is formed and comparted by the lower surface of the second solid electrolyte layer 52a, the side surface of the second diffusion rate-determining section 60, the side surface of the second spacer layer 52b, and the upper surface of the first solid electrolyte layer 52c.

In this embodiment, the first diffusion rate-determining section 58 is made of a porous ceramic having gas-permeability. The inner pumping electrode 64 exposed to the atmosphere in the first chamber 54 is composed of a cermet comprising Au: 0.5 % and Pt: 99.5 %. The second diffusion rate-determining section 60 is constructed by a gap.

NOx is measured by the sensing device 50B according to the second embodiment, while setting the voltage Vpl of the DC power source 106 connected to the main pumping cell 68 to be, for example, 450 mV (650 °C) in a fixed manner, and setting the voltage Vp2 of the DC power source 86 connected to the measuring pumping cell 84 to be, for example, 400 mV (650 °C) in a fixed manner.

The other construction and the principle of measurement of nitrogen oxides are substantially the same as those of the sensing device 50A according to the first embodiment, duplicate explanation of which will be omitted.

Now, an illustrative experiment for the sensing device 50B according to the second embodiment will be described. In this illustrative experiment, the output characteristics of the pumping current Ip2 were observed by using a measurement gas having a composition comprising oxygen: 8 %, carbon dioxide gas: 6 %, NOx: 10 to 200 ppm, and balance: nitrogen gas and saturated steam, wherein the temperature of both the first and second chambers 54, 56 was 650 °C, the pumping current Vp1 applied to the main pumping cell 68 was 450 mV, and the measuring voltage Vp2 applied to the measuring pumping cell 84 was 400 mV or 600 mV.

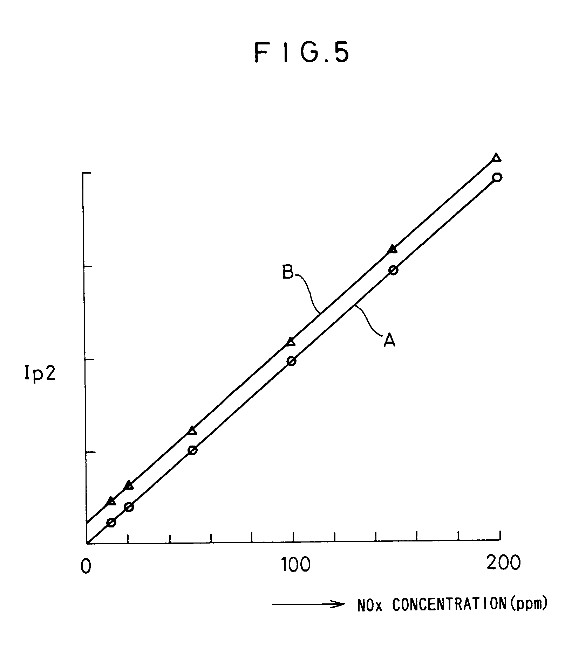

Results of the experiment are shown in FIG. 5. In FIG. 5, circles represent an output characteristic (characteristic A) obtained by adjusting the measuring voltage Vp2 at 400 mV, and triangles represent an output characteristic (characteristic B) obtained by adjusting the measuring voltage Vp2 at 600 mV.

According to the results of the experiment shown in FIG. 5, it is understood that the output characteristic (characteristic A), obtained by adjusting the measuring voltage Vp2 at 400 mV lower than the pumping voltage Vp1, forms a straight line passing through the origin, in which no offset is observed.

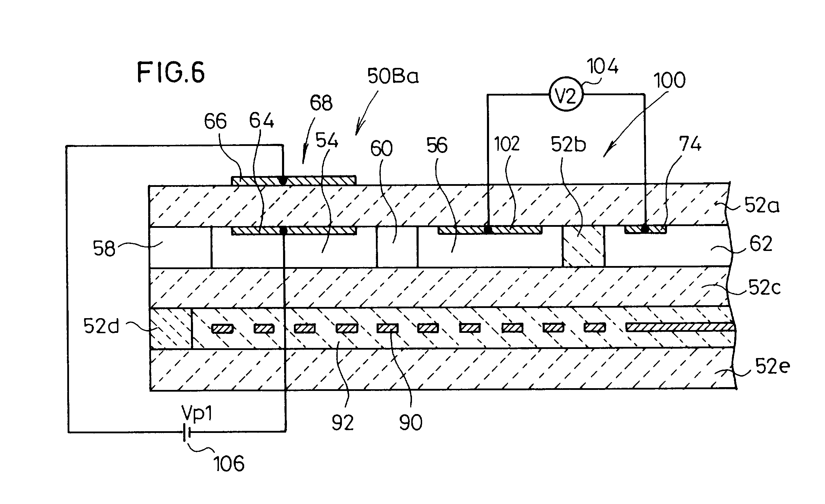

Next, a modified embodiment of the sensing device 50B according to the second embodiment will be explained with reference to FIG. 6. Components or parts corresponding to those shown in FIGs. 3 and 4 are designated by the same reference numerals, duplicate explanation of which will be omitted.

As shown in FIG. 6, a sensing device 50Ba according to this modified embodiment has approximately the same structure as that of the sensing device 50B according to the second embodiment (see FIG. 4). However, the former is different from the latter in that a measuring oxygen partial pressure-detecting cell 100 is provided in place of the measuring pumping cell 84, in the same manner as the sensing device 50Aa according to the modified embodiment of the first embodiment.

An electromotive force (electromotive force of an oxygen concentration cell) V2 is generated between the detecting electrode 102 and the reference electrode 74 of the measuring oxygen partial pressure-detecting cell 100, corresponding to a difference in oxygen concentration between an atmosphere around the detecting electrode 102 and an atmosphere around the reference electrode 74. Therefore, the partial pressure of oxygen in the atmosphere around the detecting electrode 102 is detected as a value of the voltage V2 by measuring the electromotive force (voltage) V2 by using the voltmeter 104.

In this embodiment, the offset of the electromotive force V2 generated in the measuring oxygen partial pressure-detecting cell 100 can be made zero, and it is possible to correctly measure even a minute amount of NOx as well.

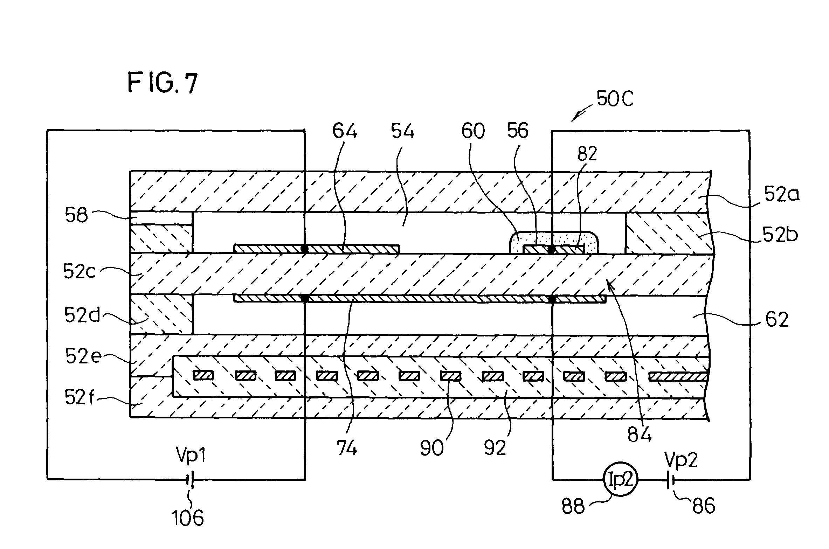

Next, a sensing device 50C according to a third embodiment will be explained with reference to FIG. 7. Components or parts corresponding to those shown in FIG. 4 are designated by the same reference numerals, duplicate explanation of which will be omitted.

As shown in FIG. 7, the sensing device 50C according to the third embodiment has approximately the same structure as that of the sensing device 50B according to the second embodiment (see FIG. 4). However, the former is different from the latter in the following points.

Namely, the sensing device 50C according to the third embodiment comprises, as a whole, six stacked solid electrolyte layers 52a to 52f composed of ceramics based on the use of oxygen ion-conductive solid electrolytes such as $ZrO_2$. First and second layers from the bottom are designated as first and second substrate layers 52f, 52e respectively. Third and fifth layers from the bottom are designated as first and second spacer layers 52d, 52b respectively. Fourth and sixth layers from the bottom are designated as first and second solid electrolyte layers 52c, 52a respectively.

A reference gas-introducing space 62 is formed and comparted between the second substrate layer 52b and the first solid electrolyte layer 52c by a lower surface of the first solid electrolyte layer 52c, an upper surface of the second substrate layer 52b, and side surfaces of the first spacer layer 52d. A reference electrode 74 is formed on a portion exposed to the reference gas-introducing space 62, of the lower surface of the first solid electrolyte layer 52c.

The sensing device 50C according to the third embodiment includes a first diffusion rate-determining section 58 formed as an slit-shaped opening. A second diffusion rate-determining section 60 is constructed by a porous $Al_2O_3$ layer or a porous $ZrO_2$ layer formed to cover the detecting electrode 82 of the measuring pumping cell 84. Therefore, the second chamber 56 corresponds to an interface between the detecting electrode 82 and the second diffusion rate-determining section 60 composed of the porous layer.

The sensing device 50C according to the third embodiment includes an inner pumping electrode 64 for constructing the main pumping cell 68, formed on a portion exposed to a first chamber 54, of the upper surface of the first solid electrolyte layer 52c, wherein the reference electrode 74 also serves as the outer pumping electrode 66 of the main pumping cell 68 described above (see FIG. 4). The inner pumping electrode 64 is composed of a cermet comprising Au: 0.5 % and Pt: 99.5 %. The detecting electrode 82 is composed of an Rh cermet.

Therefore, the pumping current Ip1 is allowed to flow in the positive direction between the reference electrode 74 and the inner pumping electrode 64 by applying a desired fixed voltage (pumping voltage) Vp1 between the inner pumping electrode 64 and the reference electrode 74 of the main pumping cell 68 by the aid of the external DC power source 106. Accordingly, the oxygen in the atmosphere in the first chamber 54 is pumped out to the reference gas-introducing space 62.

Now, an illustrative experiment for the sensing device 50C according to the third embodiment will be described. In this illustrative experiment, the output characteristic of the pumping current Ip2 flowing through the measuring pumping cell 84 was observed by using a measurement gas having a composition comprising oxygen: 20 %, NOx: 10 to 200 ppm, and balance: nitrogen gas and saturated steam, wherein the temperature of both the first and second chambers 54, 56 was 650 °C, the partial pressure of oxygen in the first chamber 54 was $10^{-8}$ atm, and the partial pressure of oxygen in the second chamber 56 (the interface between the second diffusion rate-determining section 60 and the detecting electrode 82) was $10^{-6}$ atm.

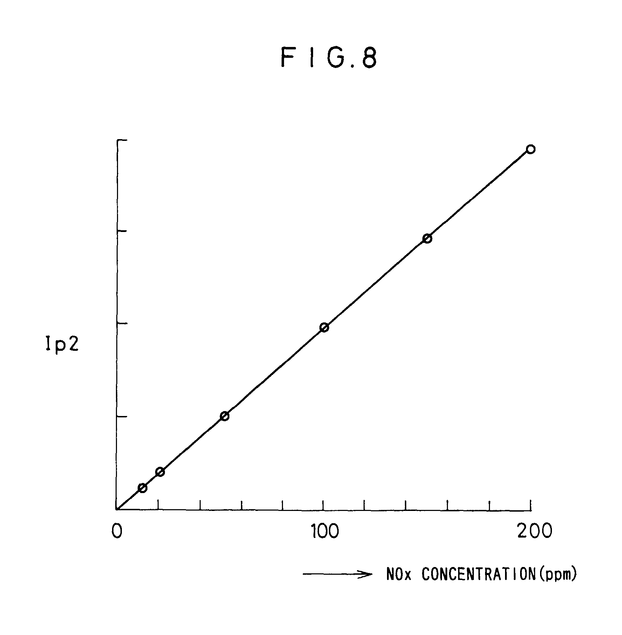

A result of the experiment is shown in FIG. 8. According to FIG. 8, it is understood that the output characteristic of the pumping current Ip2 forms a straight line passing through the origin, in which no offset is observed.

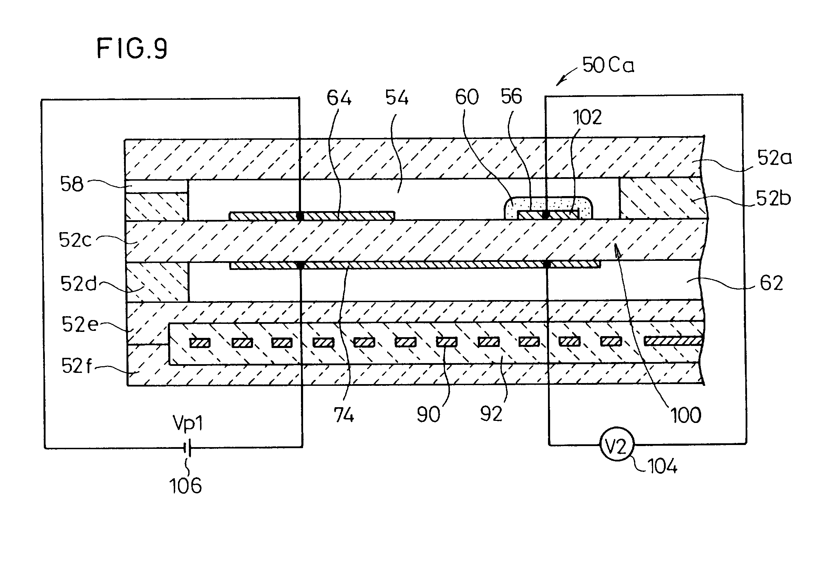

Next, a modified embodiment of the sensing device 50C according to the third embodiment will be explained with reference to FIG. 9. Components or parts corresponding to those shown in FIGs. 3 and 7 are designated by the same reference numerals, duplicate explanation of which will be omitted.

As shown in FIG. 9, a sensing device 50Ca according to this modified embodiment has approximately the same structure as that of the sensing device 50C according to the third embodiment (see FIG. 7). However, the former is different from the latter in that a measuring oxygen partial pressure-detecting cell 100 is provided in place of the measuring pumping cell 84, in the same manner as the sensing device 50Aa according to the modified embodiment of the first embodiment (see FIG. 3).

Also in this embodiment, the offset of the electromotive force V2 generated in the measuring oxygen partial pressure-detecting cell 100 can be made zero, and it is possible to correctly measure even a minute amount of NOx.

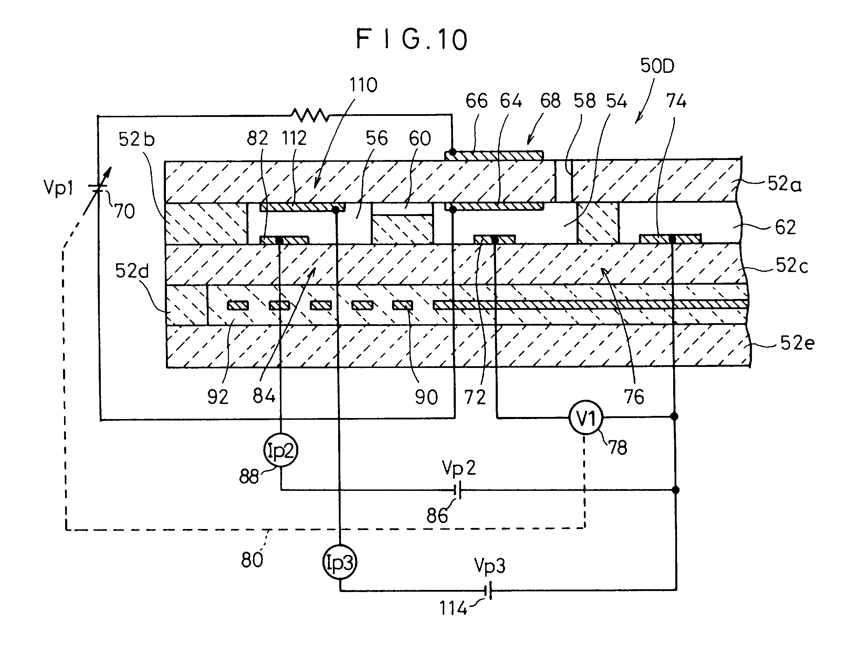

Next, a sensing device 50D according to a fourth embodiment will be explained with reference to FIG. 10. Components or parts corresponding to those shown in FIG. 2 are designated by the same reference numerals, duplicate explanation of which will be omitted.

As shown in FIG. 10, the sensing device 50D according to the fourth embodiment has approximately the same structure as that of the sensing device 50A according to the first embodiment (see FIG. 2). However, the former is different from the latter in that an auxiliary pumping cell 110 is provided for the second chamber 56.

The auxiliary pumping cell 110 comprises an auxiliary pumping electrode 112 composed of a porous cermet electrode having a flat and substantially rectangular shape and formed on a lower surface portion for forming the second chamber 56, of the lower surface of the second solid electrolyte layer 52a, the reference electrode 74, the second solid electrolyte layer 52a, the second spacer layer 52b, and the first solid electrolyte layer 52c.

The auxiliary pumping electrode 112 is constructed by using a material having a weak reducing ability or no reducing ability with respect to the NO components in the measurement gas, in the same manner as the inner pumping electrode 64 of the main pumping cell 68. In this embodiment, it is preferable that the auxiliary pumping electrode 112 is composed of, for example, a compound having the perovskite structure such as $La_3CuO_4$, a cermet comprising a ceramic and a metal having a low catalytic activity such as Au, or a cermet comprising a ceramic, a metal of the Pt group, and a metal having a low catalytic activity such as Au. Further, when an alloy comprising Au and a metal of the Pt group is used as an electrode material, it is preferable to add Au in an amount of 0.03 to 35 vol% of the entire metal components.

A desired constant voltage Vp3 is applied between the auxiliary pumping electrode 112 and the reference electrode 74 of the auxiliary pumping cell 110 by the aid of an external power source 114. Thus the oxygen in the atmosphere in the second chamber 56 can be pumped out to the reference gas-introducing space 62.

In this embodiment, the pumping voltage Vp1 of the variable power source 70 is controlled by the aid of the feedback control system 80 so that the voltage V1 which appears between the measuring electrode 72 and the reference electrode 74 of the controlling oxygen partial pressure-detecting cell 76 is, for example, 203 mV (500 °C), in the same manner as the sensing device 50A according to the first embodiment. Thus the partial pressure of oxygen in the first chamber 54 is controlled to have a predetermined value, for example, $10^{-6}$ atm. A voltage of, for example, 185 mV (600 °C) is set as the pumping voltage Vp2 applied to the measuring pumping cell 84. A voltage of, for example, 300 mV (600 °C) is set as the voltage Vp3 applied to the auxiliary pumping cell 110.

Accordingly, the partial pressure of oxygen in the atmosphere in the second chamber 56 is in a situation in which the measurement gas components (NOx) are not substantially reduced or decomposed, while giving a low value of the partial pressure of oxygen at which the measurement of the amount of the objective components is not substantially affected. In this embodiment, the change in amount of oxygen introduced into the second chamber 56 is greatly reduced as compared with the change which occurs in the measurement gas, owing to the operation of the main pumping cell 68 in the first chamber 54. Therefore, the partial pressure of oxygen in the second chamber 56 is controlled accurately and constantly.

When it is intended to control the partial pressure of oxygen in the atmosphere in the first chamber 54 to have a low value of the partial pressure of oxygen which does not substantially affect the measurement of NOx, by operating the main pumping cell 68, in other words, when the pumping voltage Vp1 of the variable power source 70 is adjusted by the aid of the feedback control system 80 so that the voltage V1 detected by the controlling oxygen partial pressure-detecting cell 76 is constant, if the oxygen concentration in the measurement gas greatly changes, for example, in a range of 0 to 20 %, then the respective partial pressures of oxygen in the atmosphere in the second chamber 56 and in the atmosphere in the vicinity of the detecting electrode 82 slightly change in ordinary cases. This phenomenon is caused probably because of the following reason. Namely, when the oxygen concentration in the measurement gas increases, the distribution of the oxygen concentration occurs in the widthwise direction and the thickness direction in the first chamber 54 over the measuring electrode 72. The distribution of the oxygen concentration changes depending on the oxygen concentration in the measurement gas.

However, in the case of the sensing device 50D according to the fourth embodiment, the auxiliary pumping cell 110 is provided for the second chamber 56 so that the partial pressure of oxygen in its internal atmosphere always has a constant low value of the partial pressure of oxygen. Accordingly, even when the partial pressure of oxygen in the atmosphere introduced from the first chamber 54 to the second chamber 56 changes depending on the oxygen concentration in the measurement gas, the partial pressure of oxygen in the atmosphere in the second chamber 56 can be always made to have a constant low value, owing to the pumping operation performed by the auxiliary pumping cell 110. As a result, the partial pressure of oxygen can be controlled to have a low value at which the measurement of NOx is not substantially affected.

As described above, the oxygen concentration in the atmosphere in the second chamber 56 is controlled to be constant by means of the auxiliary pumping cell 110. Accordingly, the pumping current Ip2 flowing through the measuring pumping cell 84 is proportional to the NOx concentration. As a result, almost all of the pumping current value Ip2 of the measuring pumping cell 84 represents the amount resulting from the reduction or decomposition of NO. Thus the pumping current value Ip2 does not depend on the oxygen concentration in the measurement gas.

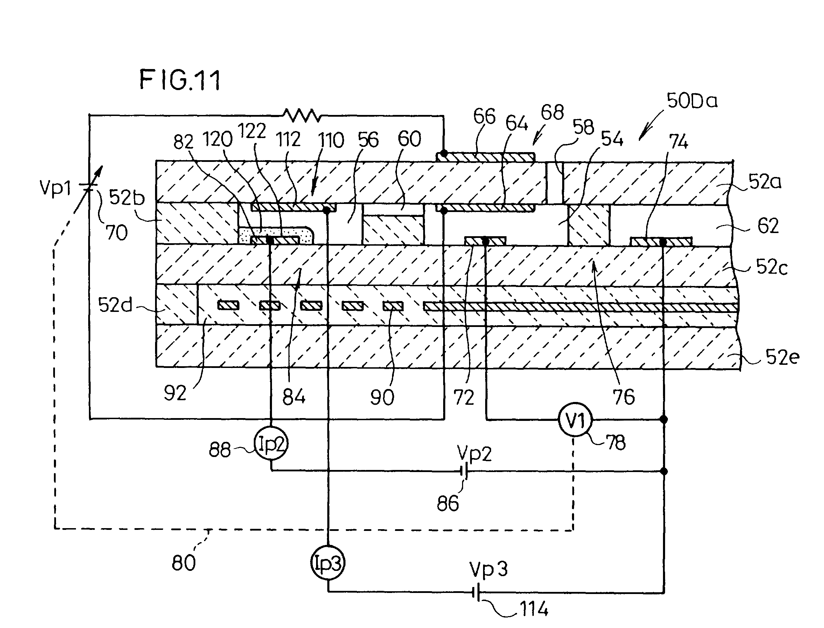

Next, a modified embodiment of the sensing device 50D according to the fourth embodiment will be explained with reference to FIG. 11. Components or parts corresponding to those shown in FIG. 10 are designated by the same reference numerals, duplicate explanation of which will be omitted.

As shown in FIG. 11, a sensing device 50Da according to this modified embodiment has approximately the same structure as that of the sensing device 50D according to the fourth embodiment (see FIG. 10). However, the former is different from the latter in that a porous $Al_2O_3$ layer or a porous $ZrO_2$ layer for constructing a third diffusion rate-determining section 120 is formed to cover the detecting electrode 82 of the measuring pumping cell 84. In this embodiment, a third chamber 122 is formed at an interface between the third diffusion rate-determining section 120 and the detecting electrode 82.

NOx flows into the measuring pumping cell 84 while being limited by the third diffusion rate-determining section 120. Under this condition, a DC power source 86 for the measuring pumping cell 84 is capable of applying a voltage Vp2 having a magnitude to give a limiting current for pumping for oxygen produced during decomposition in the measuring pumping cell 84.

Therefore, in the sensing device 50Da according to this modified embodiment, the measurement gas controlled for the partial pressure of oxygen in the second chamber 56 is introduced into the detecting electrode 82 through the third diffusion rate-determining section 120 under a predetermined diffusion resistance.

In this embodiment, the oxygen concentration in the atmosphere in the second chamber 56 is controlled to be constant by means of the auxiliary pumping cell 110. Accordingly, the pumping current Ip2 flowing through the measuring pumping cell 84 is proportional to the NOx concentration. In addition, the NOx concentration corresponds to an amount of diffusion of NOx limited by the third diffusion rate-determining section 120. Therefore, even when the oxygen concentration in the measurement gas greatly changes, the NOx concentration can be correctly measured by means of the measuring pumping cell 84 by the aid of the ammeter 88. Thus the detection sensitivity to the NOx concentration is also increased.

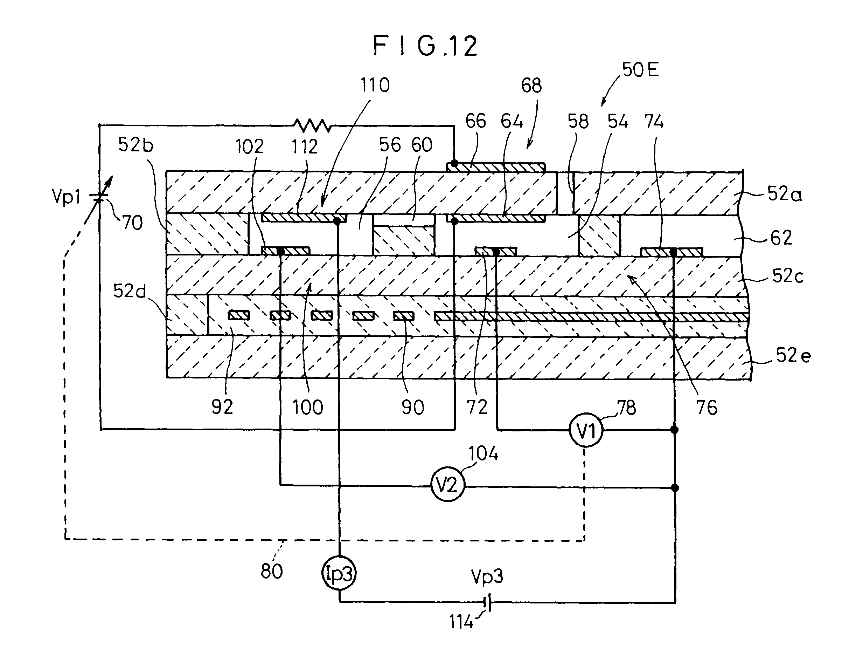

Next, a sensing device 50E according to a fifth embodiment will be explained with reference to FIG. 12. Components or parts corresponding to those shown in FIGs. 3 and 10 are designated by the same reference numerals, duplicate explanation of which will be omitted.

As shown in FIG. 12, the sensing device 50E according to the fifth embodiment has approximately the same structure as that of the sensing device 50D according to the fourth embodiment (see FIG. 10). However, the former is different from the latter in that a measuring oxygen partial pressure-detecting cell 100 is provided in place of the measuring pumping cell 84, in the same manner as the sensing device 50Aa according to the modified embodiment of the first embodiment (see FIG. 3).

An electromotive force (electromotive force of an oxygen concentration cell) V2 is generated between the detecting electrode 102 and the reference electrode 74 of the measuring oxygen partial pressure-detecting cell 100, corresponding to a difference in oxygen concentration between an atmosphere around the detecting electrode 102 and an atmosphere around the reference electrode 74. Therefore, the partial pressure of oxygen in the atmosphere around the detecting electrode 102 is detected as a value of the voltage V2 by measuring the electromotive force (voltage) V2 by using the voltmeter 104.

In this embodiment, even when the partial pressure of oxygen in the atmosphere introduced from the first chamber 54 to the second chamber 56 changes depending on the oxygen concentration in the measurement gas, the partial pressure of oxygen in the atmosphere in the second chamber 56 can be always made to have a constant low value, owing to the pumping operation performed by the auxiliary pumping cell 110. As a result, the partial pressure of oxygen can be controlled to have a low value at which the measurement of NOx is not substantially affected.

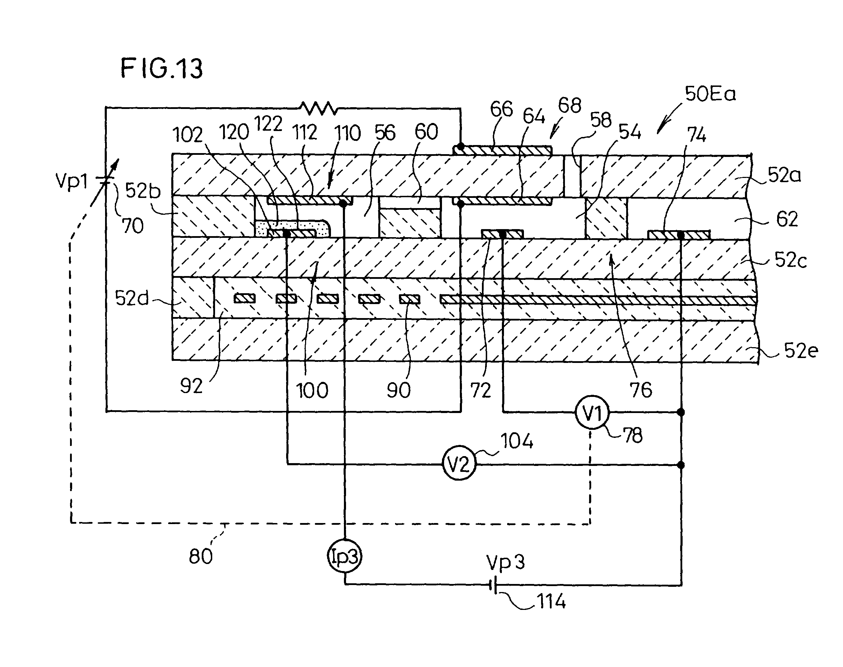

Next, a modified embodiment of the sensing device 50E according to the fifth embodiment will be explained with reference to FIG. 13. Components or parts corresponding to those shown in FIG. 12 are designated by the same reference numerals, duplicate explanation of which will be omitted.

As shown in FIG. 13, a sensing device 50Ea according to this modified embodiment has approximately the same structure as that of the sensing device 50E according to the fifth embodiment. However, the former is different from the latter in that a porous $Al_2O_3$ layer or a porous $ZrO_2$ layer for constructing a third diffusion rate-determining section 120 is formed to cover the detecting electrode 102 of the measuring oxygen partial pressure-detecting cell 100. In this embodiment, a third chamber 122 is formed at an interface between the third diffusion rate-determining section 120 and the detecting electrode 102.

In this embodiment, the measurement gas introduced into the second chamber 56 diffuses toward the detecting electrode 102 under a predetermined diffusion resistance of the third diffusion rate-determining section 120 arranged in the second chamber 56. As a result, NOx is reduced on the detecting electrode 102, and the electromotive force V2 generated between the detecting electrode 102 and the reference electrode 74 is measured by the voltmeter 104.

Also in the sensing device 50Ea according to this modified embodiment, even when the oxygen concentration in the measurement gas greatly changes, the NOx concentration can be correctly measured by means of the measuring oxygen partial pressure-detecting cell 100 by the aid of the voltmeter 104, in the same manner as the sensing device 50E according to the fifth embodiment. Thus the detection sensitivity to the NOx concentration is also increased.

According to the sensing devices concerning the first to fifth embodiments (including the respective modified embodiments), even a minute amount of NOx can be correctly measured in a state in which substantially no offset occurs, without being affected by water, carbon dioxide gas, and oxygen which co-exist in the measurement gas. Thus the present invention is extremely useful from the industrial viewpoint.

It is a matter of course that the method for measuring nitrogen oxides according to this invention is not limited to the embodiments described above, which can be constructed in other various forms within the scope of the invention.

## Claims

1. A method for measuring nitrogen oxides, comprising the steps of:

    using a main pumping means (68) including a pair of first and second pumping electrodes (64, 66), said first pumping electrode (64) being arranged in an area into which a measurement gas is introduced from external space, wherein oxygen contained in said measurement gas introduced from said external space is subjected to a pumping process on the basis of a control voltage (Vpl) applied between said pair of pumping electrodes (64, 66) so that a partial pressure of oxygen in a process atmosphere is controlled to have a predetermined value at which NO is not decomposable;
    using an electric signal-generating conversion means including a pair of first and second detecting electrodes (82, 74 or 102, 74), said first detecting electrode (82 or 102) being arranged in an area into which said measurement gas is introduced after being subjected to said pumping process performed by said main pumping means (68), wherein said electric signal-generating conversion means provides, by conversion, an electric signal (Ip2 or V2) corresponding to an amount of oxygen contained in said measurement gas after being subjected to said pumping process performed by said main pumping means (68); and
    measuring said nitrogen oxides in said measurement gas on the basis of said electric signal (Ip2 or V2) supplied from said electric signal-generating conversion means.

2. The method according to claim 1, wherein said electric signal-generating conversion means is operated as follows:

    said oxygen contained in said measurement gas after being subjected to said pumping process performed by said main pumping means (68) is subjected to a pumping process on the basis of a measuring voltage (Vp2) applied between said pair of detecting electrodes (82, 74) so that a partial pressure of oxygen in a process atmosphere is controlled to have a predetermined value at which NO is decomposable;
    a pumping current (Ip2) flowing through said electric signal-generating conversion means in accordance with said pumping process performed by said electric signal-generating conversion means is detected in a state in which said partial pressure of oxygen in said process atmosphere processed by said electric signal-generating conversion means is not less than said partial pressure of oxygen in said process atmosphere processed by said main pumping means (68); and
    said nitrogen oxides in said measurement gas are measured on the basis of said detected pumping current (Ip2).

3. The method according to claim 1, wherein said electric signal-generating conversion means is operated as follows:

    said oxygen contained in said measurement gas after being subjected to said pumping process performed by said main pumping means (68) is subjected to a pumping process on the basis of a measuring voltage (Vp2) applied between said pair of detecting electrodes (82, 74) so that a partial pressure of oxygen in a process atmosphere is controlled to have a predetermined value at which NO is decomposable;
    a pumping current (Ip2) flowing through said electric signal-generating conversion means in accordance with said pumping process performed by said electric signal-generating conversion means is detected in a state in which a level of said measuring voltage (Vp2) supplied to said electric signal-generating conversion means is not more than a level of said control voltage (Vp1) supplied to said main pumping means (68); and
    said nitrogen oxides in said measurement gas are measured on the basis of said detected pumping current (Ip2).

4. The method according to claim 1, wherein said electric signal-generating conversion means performs said pumping process for said oxygen produced by any one of or both of actions of application of a voltage sufficient to decompose said nitrogen oxides between said pair of electrodes (82, 74), and a nitrogen oxide-decomposing catalyst arranged in said electric signal-generating conversion means, on the basis of said measuring voltage (Vp2) applied between said pair of electrodes (82, 74).

5. The method according to claim 4, wherein said nitrogen oxide-decomposing catalyst is an Rh cermet.

6. The method according to claim 1, wherein said electric signal-generating conversion means is operated as follows:

    an electromotive force (V2) corresponding to a difference between said amount of oxygen contained in said measurement gas after being subjected to said pumping process performed by said main pumping means (68) and an amount of oxygen contained in a gas existing on a side of said second detecting electrode (74) is detected in a state in which said partial pressure of oxygen in a process atmosphere processed by said electric signal-generating conversion means is not less than said partial pressure of oxygen in said process atmosphere processed by said main pumping means (68); and
said nitrogen oxides in said measurement gas are measured on the basis of said detected electromotive force (V2).

7. The method according to claim 6, wherein said electric signal-generating conversion means generates said electromotive force of an oxygen concentration cell (V2) corresponding to a difference in partial pressure between oxygen produced by an action of a nitrogen oxide-decomposing catalyst arranged in said electric signal-generating conversion means and said oxygen contained in said gas existing on said side of said second detecting electrode (74).

8. The method according to any one of claims 1 to 8, wherein an electrode having a low catalytic activity on NO is used as at least one of said electrodes exposed to said spaces (54, 56) for processing said measurement gas introduced from said external space.

9. The method according to claim 8, wherein an electrode having a low catalytic activity on NO contains Au or an alloy composed of Au and an element belonging to the platinum group.

10. The method according to any one of claims 1 to 9, further comprising the steps of:

   using a concentration-detecting means (76) including a pair of first and second measuring electrodes (72, 74), said first measuring electrode (72) being arranged to be opposed to said first pumping electrode (64) of said main pumping means (68), wherein an electromotive force (V1) of an oxygen concentration cell generated corresponding to a difference between an amount of oxygen contained in said measurement gas during said pumping process performed by said main pumping means (68) and an amount of oxygen contained in a gas existing on a side of said second measuring electrode (74) is measured; and
   adjusting said control voltage (Vp1) for said main pumping means (68) on the basis of said electromotive force (V1) measured by said concentration-measuring means (76).

11. The method according to any one of claims 1 to 9, further comprising the step of using an auxiliary pumping means (110) including an auxiliary pumping electrode (112) provided in the vicinity of said first detecting electrode (82 or 102), wherein said oxygen contained in said measurement gas after being subjected to said pumping process performed by said main pumping means (68) is subjected to a pumping process on the basis of a voltage applied between said auxiliary pumping electrode (112) and said second detecting electrode (74).

12. The method according to claim 10, wherein said second measuring electrode (74) is arranged at a position exposed to a space (62) into which a reference gas is introduced.

13. The method according to either claim 10 or claim 12, wherein said second measuring electrode (74) of said concentration-detecting means (76) is combined into a common unit with said second detecting electrode (74) of said electric signal-generating conversion means including said pair of first and second detecting electrodes (82, 74 or 102, 74), said first detecting electrode (82 or 102) being arranged in an area into which said measurement gas is introduced after being subjected to said pumping process performed by said main pumping means (68), for providing, by conversion, said electric signal (Ip2 or V2) corresponding to said amount of oxygen contained in said measurement gas after being subjected to said pumping process performed by said main pumping means (68).

14. The method according to any one of claims 1 to 13, wherein:

   said main pumping means (68) includes inner and outer pumping electrodes (64, 66) formed at the inside and the outside of a first chamber (54) surrounded by substrates composed of solid electrolytes (52a, 52b, 52c), for introducing said measurement gas thereinto, and said substrate interposed between said both electrodes (64, 66); and
   said electric signal-generating conversion means includes said detecting electrode (82 or 102) formed at the inside of a second chamber (56) surrounded by substrates composed of solid electrolytes (52a, 52b, 52c), for introducing said measurement gas thereinto after being subjected to said pumping process performed by said main pumping means (68), a reference electrode (74) formed at the inside of a reference gas-introducing space (62) surrounded by substrates composed of solid electrolytes (52a, 52b, 52c or 52c, 52d, 52e), for introducing a reference gas thereinto, and said substrate interposed between said detecting electrode (82 or 102) and said reference electrode (74).

15. The method according to any one of claims 10. 12 and 13, wherein said concentration-measuring means (76) includes:

said measuring electrode (72) formed at the inside of a first chamber (54) surrounded by substrates composed of solid electrolyte (52a, 52b, 52c), for introducing said measurement gas from said external space;
a reference electrode (74) formed at the inside of a reference gas-introducing space (62) surrounded by substrates composed of solid electrolytes (52a, 52b, 52c or 52c, 52d, 52e), for introducing a reference gas thereinto; and
said substrate interposed between said measuring electrode (72) and said reference electrode (74).

16. The method according to either claim 14 or claim 15, wherein:

said measurement gas is introduced from said external space into said first chamber (54) through a first diffusion rate-determining section (58) for giving a predetermined diffusion resistance to said measurement gas; and
said measurement gas after being subjected to said pumping process performed by said main pumping means (68) is introduced into said second chamber (56) through a second diffusion rate-determining section (60) for giving a predetermined diffusion resistance to said measurement gas.

17. The method according to any one of claims 14 to 16, wherein said measurement gas in said second chamber (56) is introduced into an area of said first detecting electrode (82 or 102) through a third diffusion rate-determining section (120) for giving a predetermined diffusion resistance to said measurement gas.

# F I G.1

50A

60    54

A                                        A

56    58                    62

EP 0 791 828 A1

# F I G. 2

# FIG.3

EP 0 791 828 A1

FIG.4

# F I G.5

Ip2

B

A

0    100    200

⟶ NOx CONCENTRATION(ppm)

FIG.6

FIG.7

# F I G . 8

Ip2

0            100            200

⟶ NOx CONCENTRATION(ppm)

# FIG.9

EP 0 791 828 A1

EP 0 791 828 A1

## F I G.10

FIG.11

# FIG.12

EP 0 791 828 A1

EP 0 791 828 A1

# FIG.13

| | European Patent Office | EUROPEAN SEARCH REPORT | Application Number |
| | | | EP 97 30 1200 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | EP 0 678 740 A (NGK INSULATORS LTD) 25 October 1995<br>* column 11, line 21 - column 16, line 9 *<br>* column 21, line 7 - line 34 *<br>--- | 1-16 | G01N27/407<br>G01N27/419 |
| A | EP 0 257 842 A (NGK INSULATORS LTD) 2 March 1988<br>* claims *<br>--- | 1-17 | |
| A | US 5 217 588 A (WANG DA Y ET AL) 8 June 1993<br>* column 2, line 15 - column 4, line 10 *<br>--- | 1-17 | |
| A | US 5 034 112 A (MURASE ISAO ET AL) 23 July 1991<br>* column 1, line 63 - column 4, line 60 *<br>--- | 1-17 | |
| A | US 4 927 517 A (MIZUTANI YOSHIHIKO ET AL) 22 May 1990<br>* column 1, line 48 - column 7, line 12 *<br>--- | 1-17 | |
| A | GB 2 288 873 A (MIDDLESEX UNIVERSITY SERVICES ;COPCUTT ROBERT CHARLES (GB); MASKEL) 1 November 1995<br>* page 4 - page 6 *<br>--- | 1-17 | TECHNICAL FIELDS SEARCHED (Int.Cl.6)<br><br>G01N |
| P,A | EP 0 731 351 A (NGK INSULATORS LTD) 11 September 1996<br>* claims *<br>--- | 1-17 | |
| P,A | DE 44 39 901 A (BOSCH GMBH ROBERT) 9 May 1996<br>* column 2, line 29 - column 4, line 5 *<br>--- | 1-17 | |
| D,A | EP 0 351 960 A (FORD MOTOR COMPANY) 24 January 1990<br>* column 1 - column 4, line 28 *<br>----- | 1-17 | |

The present search report has been drawn up for all claims

| Place of search | Date of completion of the search | Examiner |
|---|---|---|
| THE HAGUE | 22 May 1997 | CALLEWAERT, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)